(19) Europäisches Patentamt
European Patent Office
Office européen des brevets

(11) Veröffentlichungsnummer: **0 004 931**
**B1**

# (12) EUROPÄISCHE PATENTSCHRIFT

(45) Veröffentlichungstag der Patentschrift: **05.08.81**

(51) Int. Cl.³: **A 01 N 43/08**, A 01 N 43/10, C 07 D 307/68, C 07 D 333/38

(21) Anmeldenummer: **79101097.8**

(22) Anmeldetag: **10.04.79**

## (54) Verwendung von Furan- und Thiophenderivaten zur Regulierung des Pflanzenwachstums.

(30) Priorität: **21.04.78 DE 2817449**

(43) Veröffentlichungstag der Anmeldung: **31.10.79 Patentblatt 79/22**

(45) Bekanntmachung des Hinweises auf die Patenterteilung: **05.08.81 Patentblatt 81/31**

(84) Benannte Vertragsstaaten: **BE CH DE FR GB IT NL**

(56) Entgegenhaltungen:
**DE-A-2 135 623**
**US-A-3 536 473**
**US-A-3 819 357**
**US-A-3 989 505**

**DERWENT JAPANESE PATENTS REPORT, vol. 6, nr. 6 (21-03-1967), London (GB) Zusammenfassung Nr. G 8291**

(73) Patentinhaber: **BAYER AG, Zentralbereich Patente, Marken und Lizenzen, D-5090 Leverkusen 1, Bayerwerk (DE)**

(72) Erfinder: **Dickoré, Karlfried, Dr., Nicolai-Hartmann-Strasse 19, D-5090 Leverkusen 1 (DE)**
Erfinder: **Lürssen, Klaus, Dr., August-Kierspel-Strasse 89, D-5060 Bergisch-Gladbach 2 (DE)**

**EP 0 004 931 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verwendung von Furan- und Thiophenderivaten zur Regulierung des Pflanzenwachstums

Die vorliegende Erfindung betrifft die Verwendung von teilweise bekannten Furan- und Thiophenderivaten als Wirkstoffe zur Hemmung des Pflanzenwuchses, zur Stimulation der pflanzlichen Ethylenbiosynthese, zur Entblätterung bei Baumwolle sowie zur Reifebeschleunigung bei Tomaten.

Es ist bereits bekannt geworden, dass bestimmte Thienylharnstoffe herbizide Wirksamkeit besitzen (vgl. DT-OS 2 510 936). Ferner wurde Synthese mehrerer 2-Amino-thiophene bzw. 2-Amino-furane in der Literatur beschrieben [vgl. Ber. 99, 1002–1007 (1966), Ber. 99, 94–100 (1966) und Ber. 99, 2712–2715 (1966)]. Weiterhin ist bekannt, dass bestimmte Acyl-thiophene als Ausgangsprodukte zur Herstellung pharmakologisch aktiver Verbindungen dienen können [vgl. Arch. Pharm. 309, 914–919 (1976)].

Es ist ausserdem bereits bekannt geworden, dass (2-Chloräthyl)-trimethylammonium-chlorid pflanzenwuchsregulierende Eigenschaften aufweist (vgl. US-Patentschrift 3 156 554). Die Wirksamkeit dieses Stoffes ist jedoch, – vor allem bei niedrigen Aufwandmengen –, nicht immer ganz befriedigend.

Ebenso ist bereits bekannt, dass 2-Halogenäthansulfinsäure, beispielsweise 2-Chloräthansulfinsäure, und ihre Derivate als Pflanzenwachstumsregulatoren verwendet werden können (vergleiche Deutsche Offenlegungsschrift 2 110 773). Auch ihre Wirkung ist jedoch vor allem bei niedrigen Aufwandmengen nicht immer ganz befriedigend.

Darüber hinaus ist bekannt, dass bestimmte 2-Aminotetramethylen-thiophen-Derivate, wie beispielsweise 2-Acetamido-3-carbäthoxy-4,5-tetramethylen-thiophen und 2-Isopropylcarbonyl-amino-3-carbäthoxy-4,5-tetramethylen-thiophen, als Pflanzenwachstumsregulatoren geeignet sind (vgl. DT-OS 2 627 935). Die Wirkung dieser Stoffe ist allerdings ebenfalls nicht immer ausreichend.

Es wurde nun gefunden, dass die teilweise bekannten Heterocyclen der Formel

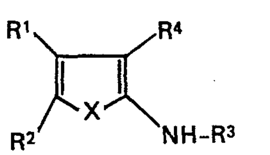

(I)

in welcher

$R^1$ für Alkyl mit 1 bis 6 Kohlenstoffatomen oder für gegebenenfalls durch Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen, Alkylthio mit 1 bis 4 Kohlenstoffatomen, Alkylthio mit 1 bis 4 Kohlenstoffatomen, Alkylsulfonyl mit 1 bis 4 Kohlenstoffatomen und/oder Nitro substituiertes Phenyl oder Naphtyl steht,

$R^2$ für Wasserstoff, Alkyl mit 1 bis 6 Kohlenstoffatomen oder für gegebenenfalls durch Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen, Alkylthio mit 1 bis 4 Kohlenstoffatomen, Alkylsulfonyl mit 1 bis 4 Kohlenstoffatomen und/oder Nitro substituiertes Phenyl oder Naphthyl steht,

$R^3$ für Wasserstoff oder die Gruppierung

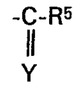 steht

in welcher

Y für Sauerstoff oder Schwefel steht und

$R^5$ für Alkyl mit 1 bis 6 Kohlenstoffatomen, Halogenalkyl mit 1 bis 4 Kohlenstoffatomen und 1 bis 5 Halogenatomen, Alkoxylalkyl mit 1 bis 4 Kohlenstoffatomen im Alkylteil und 1 bis 4 Kohlenstoffatomen im Alkoxyteil, gegebenenfalls im Aryloxyteil durch Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen, Alkylthio mit 1 bis 4 Kohlenstoffatomen, Alkylsulfonyl mit 1 bis 4 Kohlenstoffatomen und/oder Nitro substituiertes Aryloxyalkyl mit 6 oder 10 Kohlenstoffatomen im Aryloxyteil und 1 bis 4 Kohlenstoffatomen im Alkylteil, für Alkoxy mit 1 bis 4 Kohlenstoffatomen, gegebenenfalls durch Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen, Alkylthio mit 1 bis 4 Kohlenstoffatomen, Alkylsulfonyl mit 1 bis 4 Kohlenstoffatomen und/oder Nitro substituiertes Phenyl oder Naphthyl, für gegebenenfalls durch Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen, Alkylthio mit 1 bis 4 Kohlenstoffatomen, Alkylsulfonyl mit 1 bis 4 Kohlenstoffatomen und/oder Nitro substituiertes Phenoxy oder Naphthoxy, für Monoalkylamino mit 1 bis 4 Kohlenstoffatomen, Dialkylamino mit 1 bis 4 Kohlenstoffatomen in jeder Alkylgruppe, Alkenylamino mit bis zu 4 Kohlenstoffatomen, Dialkenylamino mit bis zu 4 Kohlenstoffatomen in jeder Alkenylgruppe, für gegebenenfalls durch Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen, Alkylthio mit 1 bis 4 Kohlenstoffatomen, Alkylsulfonyl mit 1 bis 4 Kohlenstoffatomen und/oder Nitro substituiertes Arylamino mit 6 oder 10 Kohlenstoffatomen, für einen 5- bis 7-gliedrigen gesättigten heterocyclischen Ring, der über Stickstoff gebunden ist, und in dem 1 bis 3 Kohlenstoffatome ersetzt sein können durch Sauerstoff, Schwefel, $-SO_2-$, -NH- und/oder $-N-(CH_3)-$, oder für die Gruppierung der Formel

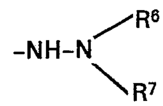

steht,
in welcher
$R^6$ für Wasserstoff oder Alkyl mit 1 bis 4 Kohlenstoffatomen steht,
$R^7$ für Wasserstoff oder Alkyl mit 1 bis 4 Kohlenstoffatomen steht und
$R^6$ und $R^7$ gemeinsam mit dem angrenzenden Stickstoffatom für einen 5- bis 7-gliedrigen gesättigten heterocyclischen Ring stehen, in dem 1 bis 3 Kohlenstoffatome ersetzt sein können durch Sauerstoff, Schwefel, $-SO_2-$, -NH- und (oder) $-N(CH_3)-$,
$R^4$ für Cyano oder die Gruppierung $-COR^8$ steht, in welcher
$R^8$ für Alkoxy mit 1 bis 4 Kohlenstoffatomen oder Amino steht, und
X für Sauerstoff oder Schwefel steht,
zur Hemmung des Pflanzenwuchses, zur Stimulation der pflanzlichen Ethylenbiosynthese, zur Entblätterung bei Baumwolle sowie zur Reifebeschleunigung bei Tomaten verwendet werden können.

Überraschenderweise zeigen die erfindungsgemäss verwendbaren Heterocyclen der Formel (I) bei den angegebenen Indikationen eine bessere pflanzenwachstumsregulierende Wirksamkeit als das aus dem Stand der Technik bekannte (2-Chloräthyl)-trimethylammonium-chlorid und die ebenfalls bekannte 2-Chloräthan-sulfinsäure, welche anerkannt gut wirksame Stoffe gleicher Wirkungsrichtung sind. Sie besitzen ausserdem bei den angegebenen Indikationen eine höhere Aktivität als das 2-Acetamido-3-carbäthoxy-4,5-tetramethylen-thiophen und das 2-Isopropyl-carbonyl-amino-3-carbäthyoxy-4,5-tetramethylen-thiophen, welches die chemisch ähnlichsten vorbekannten Stoffe gleicher Wirkungsart sind. Die erfindungsgemäss verwendbaren Stoffe stellen somit eine wertvolle Bereicherung der Technik dar.

Besonders bevorzugt verwendbar sind solche Verbindungen der Formel (I), in denen $R^1$ für Methyl, Äthyl, n-Propyl, Isopropyl, n-Butyl, sek.-Butyl, tert.-Butyl oder gegebenenfalls auch Fluor, Chlor, Brom, Methyl, Äthyl, n-Propyl, Isopropyl, Trifluormethyl, Trichlormethyl, Methoxy, Äthoxy, Methylthio, Äthylthio, Methylsulfonyl, Äthylsulfonyl und/oder Nitro substituiertes Phenyl oder Naphthyl steht, $R^2$ für Wasserstoff, Methyl, Äthyl, n-Propyl, Isopropyl, n-Butyl, sek.-Butyl, tert.-Butyl oder gegebenenfalls durch Fluor, Chlor, Brom, Methyl, Äthyl, n-Propyl, Isopropyl, Trifluormethyl, Trichlormethyl, Methoxy, Äthoxy, Methylthio, Äthylthio, Methylsulfonyl, Äthylsulfonyl und/oder Nitro substituiertes Phenyl oder Naphthyl steht, $R^3$ für Wasserstoff oder die Gruppierung

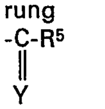

steht, in welcher Y für Sauerstoff oder Schwefel steht und $R^5$ für Methyl, Äthyl, n-Propyl, Isopropyl, n-Butyl, sek.-Butyl, tert.-Butyl, Chlormethyl, Dichlormethyl, Trichlormethyl, Chloräthyl, Dichloräthyl, Trifluormethyl, Methoxymethyl, Äthoxymethyl, Methoxyäthyl, Äthoxyäthyl, gegebenenfalls im Phenoxyteil durch Fluor, Chlor, Brom, Methyl, Äthyl, Trifluormethyl, Trichlormethyl, Methoxy, Äthoxy, Methylthio, Äthylthio, Methylsulfonyl, Äthylsulfonyl und/oder Nitro substituiertes Phenoxyalkyl mit 1 oder 2 Kohlenstoffatomen im Alkylteil steht, und $R^5$ ferner für Methoxy, Äthoxy, n-Propoxy, Isopropoxy, n-Butoxy, sek.-Butoxy, tert.-Butoxy, gegebenenfalls durch Fluor, Chlor, Brom, Methyl, Äthyl, n-Propyl, Isopropyl, n-Butyl, sek.-Butyl, tert.-Butyl, Trifluormethyl, Trichlormethyl, Methoxy, Äthoxy, Methylthio, Äthylthio, Methylsulfonyl, Äthylsulfonyl und/oder Nitro substituiertes Phenyl oder Naphthyl steht, und $R^5$ weiterhin für gegebenenfalls durch Fluor, Chlor, Brom, Methyl, Äthyl, n-Propyl, Isopropyl, n-Butyl, sek.-Butyl, tert.-Butyl, Trifluormethyl, Trichlormethyl, Methoxy, Äthoxy, Methylthio, Äthylthio, Methylsulfonyl, Äthylsulfonyl und/oder Nitro substituiertes Phenoxy oder Naphthoxy steht, und $R^5$ ferner für Methylamino, Äthylamino- n-Propylamino, Isopropylamino, n-Butylamino, Dimethylamino, Diäthylamino, Di-n-propylamino, Allylamino, Diallylamino oder für gegebenenfalls durch Fluor, Chlor, Brom, Methyl, Äthyl, N-Propyl, Isopropyl, n-Butyl, sek.-Butyl, tert.-Butyl, Trifluormethyl, Trichlormethyl, Methoxy, Äthoxy, Methylthio, Äthylthio, Methylsulfonyl, Alkylsulfonyl und/oder Nitro substituiertes Phenylamino steht, und $R^5$ weiterhin für die jeweils über Stickstoff gebundenen heterocyclischen Reste Morpholinyl, Thiomorpholinyl, Pyrrolidinyl, Piperidinyl, Piperazinyl und Hexahydroazepinyl steht, und weiterhin $R^5$ für die Gruppierung

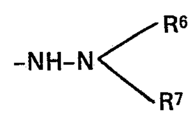

steht, in welcher $R^6$ für Wasserstoff, Methyl oder Äthyl steht, $R^7$ für Wasserstoff, Methyl oder Äthyl steht und $R^6$ und $R^7$ gemeinsam mit dem angrenzenden Stickstoffatom für Morpholinyl, Thiomorpholinyl, Pyrrolidinyl, Piperidinyl, Piperazinyl und Hexahydroazepinyl stehen, $R^4$ für Cyano oder die Gruppierung $-COR^8$ steht, in welcher $R^8$ für Methoxy, Äthoxy, n-Propoxy, Isopropoxy, n-Butoxy oder Amino steht, und X für Sauerstoff oder Schwefel steht.

Als Beispiele für Verbindungen der Formel (I) seien zusätzlich zu den in den Herstellungsbeispielen aufgeführten Stoffen im einzelnen genannt.

2-Amino-3-methoxycarbonyl-4-methyl-5-phenyl-thiophen
2-Amino-3-n-propoxycarbonyl-4-methyl-5-phenyl-thiophen
2-Amino-3-n-butoxycarbonyl-4-methyl-5-phenyl-thiophen
2-Amino-3-methoxycarbonyl-4-methyl-5-phenyl-furan
2-Amino-3-n-propoxycarbonyl-4-methyl-5-phenyl-furan
2-Amino-3-n-butoxycarbonyl-4-methyl-5-phenyl-furan
2-Amino-3-cyano-4-methyl-thiophen
2-Amino-3-cyano-4-methyl-furan
2-Phenyl-carbonyl-amino-3-äthoxycarbonyl-4,5-dimethylthiophen
2-(4-Chlorphenylcarbonyl)-amino-3-äthoxycarbonyl-4,5-dimethyl-thiophen
2-(4-Methylphenylcarbonyl)-amino-3-äthoxycarbonyl-4,5-dimethyl-thiophen
1-(3-Cyano-4-isopropyl-5-methyl-thienyl-(2)-3-methyl-harnstoff
1-(3-Cyano-4-tert.-butyl-5-methyl-thienyl-(2)-3,3-dimethyl-harnstoff
2-Amino-3-aminocarbonyl-4,5-dimethyl-thiophen
2-Acetylamino-3-aminocarbonyl-4,5-dimethylthiophen
2-Isobutyrylamino-3-aminocarbonyl-4,5-dimethyl-thiophen
4-tert.-Butyl-3-cyano-2-äthoxyacetylamino-5-methyl-furan

Die erfindungsgemäss verwendbaren Heterocyclen der Formel (I) sind teilweise bekannt [vgl. DT-OS 25 10 936, Ber. 99, 94–100 (1966), Ber. 99, 1002–1007 (1966), Ber. 99, 2712–2715 (1966) und Arch. Pharm. 309, 914–919 (1976).].

Einzelne der erfindungsgemäss verwendbaren Wirkstoffe sind neu, sie können jedoch nach im Prinzip bekannten Verfahren in einfacher Weise hergestellt werden. So erhält man Heterocyclen der Formel (I), indem man

a) Ketoverbindungen der Formel

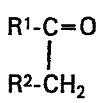

(II)

in welcher
$R^1$ und $R^2$ die oben angegebene Bedeutung haben,
mit Cyanoverbindungen der Formel

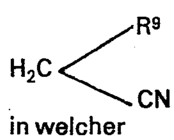

(III)

in welcher
$R^9$ für Cyano oder den Rest $-COOR^{10}$ steht, in welchem
$R^{10}$ für Alkyl mit 1–4 Kohlenstoffatomen steht,

und mit Schwefel in Gegenwart eines inerten organischen Lösungsmittels, wie Äthanol oder Dimethylformamid, sowie in Gegenwart einer Base, wie Triäthylamin oder Morpholin, bei Temperaturen zwischen 20°C und 80°C umsetzt,
oder indem man

b) Äthylene der Formel

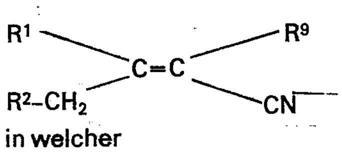

in welcher
$R^1$, $R^2$ und $R^9$ die oben angegebene Bedeutung haben, mit Schwefel in Gegenwart eines inerten organischen Lösungsmittels, wie Äthanol oder Dimethylformamid, sowie in Gegenwart einer Base, wie Triäthylamin oder Morpholin, bei Temperaturen zwischen 20°C und 80°C umsetzt,
oder indem man

c) Ketoverbindungen der Formel

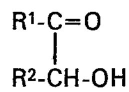

(V)

in welcher
$R^1$ und $R^2$ die oben angegebene Bedeutung, haben, mit Cyanoverbindungen der Formel (III) in Gegenwart eines inerten organischen Lösungsmittels, wie Äthanol oder Dimethylformamid, sowie in Gegenwart einer Base, wie Triäthylamin oder Morpholin, bei Temperaturen zwischen 20°C und 50°C umsetzt,
oder indem man

d) gemäss Verfahrensvarianten (a), (b) und (c) herstellbare Verbindungen der Formel

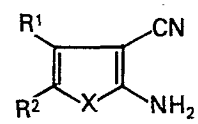

(VI)

in welcher
$R^1$, $R^2$ und X die oben angegebene Bedeutung haben, mit starken Säuren, wie Schwefelsäure, gegebenenfalls in Gegenwart eines inerten Verdünnungsmittels bei Temperaturen zwischen 80°C und 120°C in die entsprechenden 3-Aminocarbonyl-Derivate überführt,
oder indem man

e) gemäss Verfahrensvarianten (a) – (d) herstellbare Verbindungen der Formel

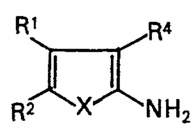

(VII)

in welcher
$R^1$, $R^2$, $R^4$ und X die oben angegebene Bedeutung haben, mit Halogenverbindungen der Formel

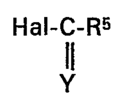

(VIII)

in welcher
$R^5$ und Y die oben angegebene Bedeutung haben und

Hal für Chlor oder Brom steht,

in Gegenwart eines inerten organischen Lösungsmittels, wie Toluol oder Dimethylformamid, sowie gegebenenfalls in Gegenwart eines säurebindenden Mittels, wie Pyridin oder Triäthylamin, bei Temperaturen zwischen 0°C und 120°C, vorzugsweise zwischen 20°C und 100°C umsetzt,
oder indem man

f) gemäss Verfahrensvarianten (a) – (d) herstell-

bare Verbindungen der Formel

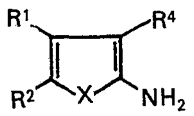

(VII)

in welcher
$R^1$, $R^2$, $R^4$ und X die oben angegebene Bedeutung haben, mit Anhydriden der Formel

$(R^{11}\text{-CO})_2O$ (IX)

in welcher
$R^{11}$ für einen der für $R^5$ genannten Alkyl-, Halogenalkyl-, Alkenyl- oder gegebenenfalls substituierten Arylreste steht,
gegebenenfalls in Gegenwart eines organischen Lösungsmittels, wie z.B. der dem Anhydrid zugrunde liegenden Säure, bei Temperaturen zwischen 20°C und 150°C, vorzugsweise zwischen 60°C und 100°C umsetzt,
oder indem man
g) gemäss Verfahrensvarianten (a) – (d) herstellbare Verbindungen der Formel

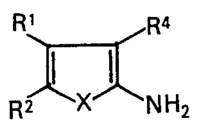

(VII)

in welcher
$R^1$, $R^2$, $R^4$ und X die oben angegebene Bedeutung haben, mit Isocyanaten bzw. Isothiocyanaten der Formel

$R^{11}$-NCY (X)

in welcher
$R^{11}$ und Y die oben angegebene Bedeutung haben, in Gegenwart eines inerten organischen Lösungsmittels, wie Toluol, bei Temperaturen zwischen 0°C und 120°C, vorzugsweise zwischen 20°C und 50°C umsetzt,
oder indem man
h) gemäss Verfahrensvarianten (a) – (d) herstellbare Verbindungen der Formel

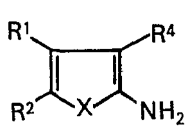

(VII)

in welcher
$R^1$, $R^2$, $R^4$ und X die oben angegebene Bedeutung haben, mit Phosgen oder Thiophosgen in Gegenwart eines inerten organischen Lösungsmittels, wie Äthylenchlorid, bei Temperaturen zwischen 40°C und 120°C, vorzugsweise zwischen 60°C und 100°C umsetzt und die dabei entstehenden Isocyanate bzw. Isothiocyanate der Formel

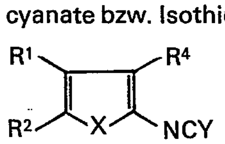

(XI)

in welcher
$R^1$, $R^2$, $R^4$, X und Y die oben angegebene Bedeutung haben, mit Verbindungen der Formel

H-$R^{12}$ (XII)

in welcher
$R^{12}$ für einen der für $R^5$ genannten Alkoxy-, gegebenenfalls im Aroxyteil substituierten Aroxyalkoxy-, gegebenenfalls substituierten Aryloxy-, Monoalkylamino-, Dialkylamino-, Alkenylamino-, Dialkenylamino-, gegebenenfalls substituierten Arylaminoreste, für einen für $R^5$ genannten 5- bis 7-gliedrigen über Stickstoff gebundenen heterocyclischen Ring oder für die Gruppierung

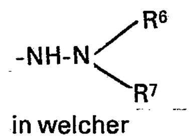

in welcher
$R^6$ und $R^7$ die oben angegebene Bedeutung haben,
gegebenenfalls in Gegenwart eines inerten organischen Lösungsmittels, wie Toluol, bei Temperaturen zwischen 0°C und 120°C, vorzugsweise 20°C und 50°C umsetzt.

Die als Ausgangsprodukte bzw. Reaktionskomponenten benötigten Verbindungen der Formeln (II), (III), (IV), (V), (VIII), (IX), (X) und (XII) sind bekannt oder lassen sich nach im Prinzip bekannten Verfahren herstellen.

Weitere Einzelheiten zur Synthese der erfindungsgemäss verwendbaren Heterocyclen sind den Herstellungsbeispielen zu entnehmen.

Die erfindungsgemäss verwendbaren Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, dass ein Wirkstoff eine oder auch mehrere verschiedenartige Wirkungen auf Pflanzen ausüben kann. Die Wirkungen der Stoffe hängen im wesentlichen ab von dem Zeitpunkt der Anwendung, bezogen auf das Entwicklungsstadium des Samens oder der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in gewünschter Weise positiv beeinflussen.

Pflanzenwuchsregulierende Stoffe können zum Beispiel zur Hemmung des vegetativen Pflanzenwachstums eingesetzt werden. Eine derartige Wuchshemmung ist unter anderem bei Gräsern von wirtschaftlichem Interesse, denn durch eine Dämpfung des Graswachstums kann z.B. die Häufigkeit der Grasschnitte in Ziergärten, Park- und Sportanlagen oder an Strassenrändern reduziert werden. Von Bedeutung ist auch die Hemmung des Wuchses von krautigen und holzigen Pflanzen an Strassenrändern und in der Nähe von Überlandleitungen oder ganz allgemein in Bereichen, in denen ein starker Bewuchs unerwünscht ist.

Wichtig ist auch die Anwendung von Wachstumsregulatoren zur Hemmung des Längenwachstums bei Getreide, denn durch eine Halmverkürzung wird die Gefahr des Umknickens («Lagerns») der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt.

Eine Hemmung des vegetativen Wachstums erlaubt bei vielen Kulturpflanzen eine dichtere Anpflanzung der Kultur, so dass ein Mehrertrag bezogen auf die Bodenfläche erzielt werden kann. Ein weiterer Mechanismus der Ertragssteigerung mit Wuchshemmern beruht darauf, dass die Nährstoffe in stärkerem Masse der Blüten- und Fruchtbildung zugute kommen, während das vegetative Wachstum eingeschränkt wird.

Unter dem Einfluss von Wachstumsregulatoren kann der Blattbestand von Pflanzen so gesteuert werden, dass ein Entblättern der Pflanzen zu einem gewünschten Zeitpunkt erreicht wird. Eine derartige Entlaubung ist von Interesse, um eine mechanische Beerntung, z.B. bei Wein oder Baumwolle, zu erleichtern oder um die Transpiration zu einem Zeitpunkt herabzusetzen, an dem die Pflanze verpflanzt werden soll.

Mit Wachstumsregulatoren lässt sich ferner eine Beschleunigung oder auch eine Verzögerung der Reife des Erntegutes vor oder nach der Ernte erreichen. Dieses ist von besonderem Vorteil, weil sich dadurch eine optimale Anpassung an die Bedürfnisse des Marktes herbeiführen lässt. Weiterhin können Wachstumsregulatoren in manchen Fällen die Fruchtausfärbung verbessern. Darüberhinaus kann mit Hilfe von Wachstumsregulatoren auch eine zeitliche Konzentrierung der Reife erzielt werden. Damit werden die Voraussetzungen dafür geschaffen, dass z.B. bei Tabak, Tomaten oder Kaffee, eine vollständige mechanische oder manuelle Beerntung in nur einem Arbeitsgang vorgenommen werden kann.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoff sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau oder organische Farbstoffe, wie Alizarin-, Azol-Metallphthalocyanifarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in den Formulierungen in Mischung mit anderen bekannten Wirkstoffen vorliegen, wie Fungizide, Insektizide; Akarizide und Herbizide, sowie in Mischung mit Düngemitteln und anderen Wachstumsregulatoren.

Die Wirkstoffe können als solche, in Form ihrer Formulierungen oder der daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Giessen, Verspritzen, Versprühen, Verstreuen, Verstäuben, Verschäumen, Begasen usw. Es ist ferner möglich, die Wirkstoffe nach dem Ultra-Low-Volume-Verfahren auszubringen, Pflanzen oder Pflanzenteile mit der Wirkstoffzubereitung oder dem Wirkstoff selbst zu bestreichen oder die Wirkstoffzubereitung oder den Wirkstoff selbst in den Boden zu injizieren. Es kann auch das Saatgut der Pflanze behandelt werden.

Die Wirkstoffkonzentrationen können in einem grösseren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, dass die Anwendung der Wachstumsregulatoren in einem bevorzugten Zeitraum vorgenommen wird, dessen genaue Abgrenzung sich nach den klimatischen und vegetativen Gegebenheiten richtet.

In den nachfolgenden Beispielen wird die Aktivität der erfindungsgemässen Stoffe dargestellt.

Beispiel A

Wuchshemmung bei Gras (Festuca pratensis)

Lösungsmittel:

30 Gewichtsteile Dimethylformamid

Emulgator:

1 Gewichtsteil polyoxyäthylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gras (Festuca pratensis) wird in 7 cm x 7 cm grossen Kunststofftöpfen angezogen und bei einer Wuchshöhe von ca. 5 cm mit den Wirkstoffzubereitungen bis zum Abtropfen besprüht. Nach 3 Wochen wird der Zuwachs gemessen und die Wuchshemmung in Prozent des Zuwachses der unbehandelten Kontrollpflanzen berechnet. Es bedeuten 100 % den Stillstand des Wachstums und 0 % ein Wachstum entsprechend dem der Kontrollpflanzen.

Wirkstoffe, Konzentration und Resultate gehen aus der nachfolgenden Tabelle hervor:

Tabelle A
Wuchshemmung bei Gras (Festuca pratensis)

| Wirkstoff | Wirkstoff-konzentration in % | Wuchs-hemmung in % |
|---|---|---|
| – (Kontrolle) | — | — |
| 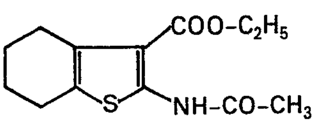 (bekannt) | 0,05 | 0 |
| 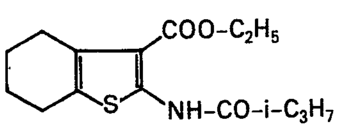 (bekannt) | 0,05 | 5 |
| 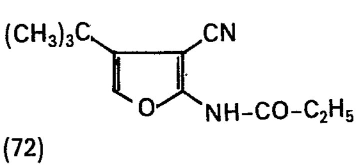 (72) | 0,05 | 40 |
| 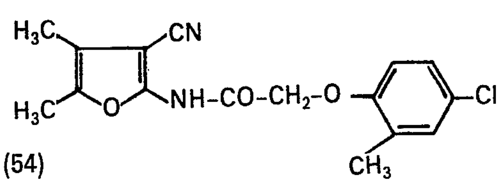 (54) | 0,05 | 25 |

Tabelle A (Fortsetzung)
Wuchshemmung bei Gras (Festuca pratensis)

| Wirkstoff | Wirkstoff-konzentration in % | Wuchs-hemmung in % |
|---|---|---|
| 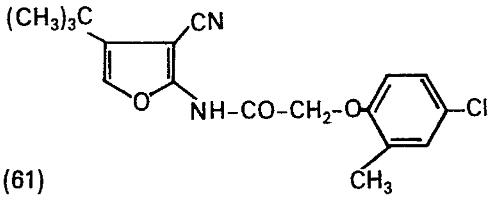 (61) | 0,05 | 30 |
| 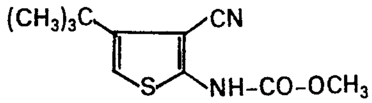 (30) | 0,05 | 45 |
| 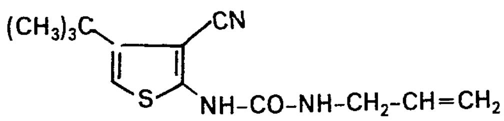 (34) | 0,05 | 100 |
| 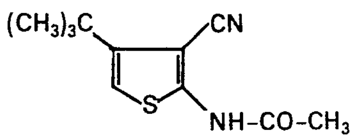 (23) | 0,05 | 45 |

Beispiel B

Reifebeschleunigung bei Tomaten

Lösungsmittel:
30 Gewichtsteile Dimethylformamid
Emulgator:
1 Gewichtsteil Polyoxyäthylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Im Freiland werden Tomaten in üblicher Weise angezogen bis etwa die Hälfte der Früchte rot gefärbt ist. In diesem Stadium werden die Pflanzen tropfnass mit den Wirkstoffzubereitungen besprüht. Nach 2 Wochen wird die Ausfärbung der Früchte bonitiert und durch Kennzahlen von 0 bis 3 bezeichnet, welche folgende Bedeutung haben:

0 = keine Reifebeschleunigung (wie unbehandelte Kontrolle)
1 = geringe Reifebeschleunigung
2 = mittlere Reifebeschleunigung
3 = starke Reifebeschleunigung

Wirkstoffe, Wirkstoffkonzentrationen und Resultate gehen aus der nachfolgenden Tabelle hervor.

Tabelle B
Reifebeschleunigung bei Tomaten

| Wirkstoff | Wirkstoff-konzentration in % | Reife-beschleuni-gung |
|---|---|---|
| — (Kontrolle) | — | 0 |
| $Cl-CH_2-CH_2-SO-OH$ (bekannt) | 0,2 | 1 |

Tabelle B
Reifebeschleunigung bei Tomaten

| Wirkstoff | Wirkstoff-konzentration in % | Reife-beschleuni-gung |
|---|---|---|
| 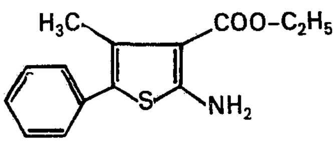 (1) | 0,2 | 2 |

Beispiel C

Wuchshemmung bei Gerste

Lösungsmittel:

30 Gewichtsteile Dimethylformamid

Emulgator:

1 Gewichtsteil Polyoxyäthylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gerstepflanzen werden im Gewächshaus bis zum 2-Blatt-Stadium angezogen. In diesem Stadium werden die Pflanzen tropfnass mit den Wirkstoffzubereitungen besprüht. Nach 3 Wochen wird bei allen Pflanzen der Zuwachs gemessen und die Wuchshemmung in % des Zuwachses der Kontrollpflanzen berechnet. Es bedeuten 100 % den Stillstand des Wachstums und 0 % ein Wachstum entsprechend dem der Kontrollpflanzen.

Wirkstoffe, Wirkstoffkonzentrationen und Resultate gehen aus der nachfolgenden Tabelle hervor:

Tabelle C
Wuchshemmung bei Gerste

| Wirkstoff | Wirkstoff-konzentration in % | Wuchs-hemmung in % |
|---|---|---|
| — (Kontrolle) | — | 0 |
| $Cl-CH_2-CH_2\overset{\oplus}{N}(CH_3)_3\overset{\ominus}{Cl}$ (bekannt) | 0,05 | 40 |
| 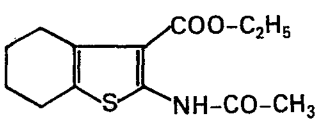 (bekannt) | 0,05 | 15 |
| 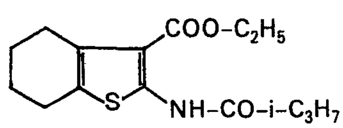 (bekannt) | 0,05 | 5 |
| 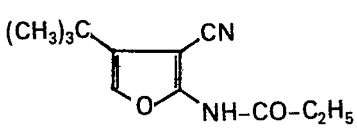 (72) | 0,05 | 95 |

Tabelle C (Fortsetzung)
Wuchshemmung bei Gerste

| Wirkstoff | Wirkstoff-konzentration in % | Wuchs-hemmung in % |
|---|---|---|
| 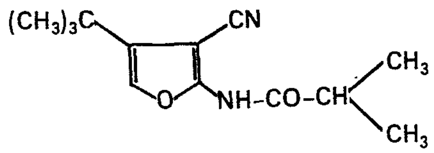 (57) | 0,05 | 55 |
| 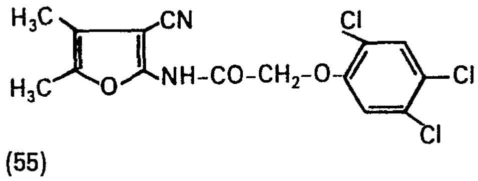 (55) | 0,05 | 50 |
| 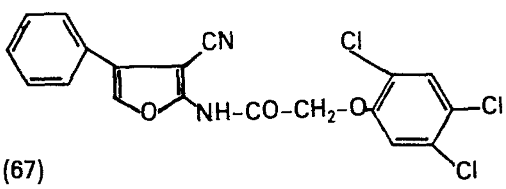 (67) | 0,05 | 60 |

Beispiel D

Wuchshemmung bei Weizen

Lösungsmittel:

30 Gewichtsteile Dimethylformamid

Emulgator:

1 Gewichtsteil Polyoxyäthylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Weizenpflanzen werden im Gewächshaus bis zum 2-Blatt-Stadium angezogen. In diesem Stadium werden die Pflanzen tropfnass mit den Wirkstoffzubereitungen besprüht. Nach 3 Wochen wird bei allen Pflanzen der Zuwachs gemessen und die Wuchshemmung in % des Zuwachses der Kontrollpflanzen berechnet. Es bedeuten 100 % den Stillstand des Wachstums und 0 % ein Wachstum entsprechend dem der Kontrollpflanzen.

Wirkstoffe, Wirkstoffkonzentrationen und Resultate gehen aus der nachfolgenden Tabelle hervor:

Tabelle D
Wuchshemmung bei Weizen

| Wirkstoff | Wirkstoff-konzentration in % | Wuchs-hemmung in % |
|---|---|---|
| — (Kontrolle) | — | 0 |
| $Cl-CH_2-CH_2-\overset{\oplus}{N}(CH_3)_3\ \overset{\ominus}{Cl}$ (bekannt) | 0,05 | 45 |
| 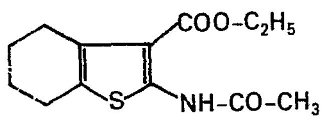 (bekannt) | 0,05 | 5 |

Tabelle D
Wuchshemmung bei Weizen

| Wirkstoff | Wirkstoff-konzentration in % | Wuchs-hemmung in % |
|---|---|---|
| 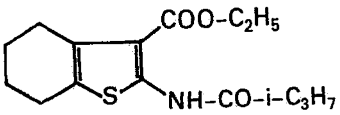 (bekannt) | 0,05 | 0 |
| 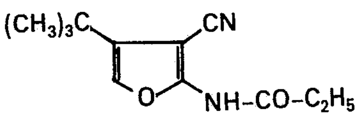 (72) | 0,05 | 85 |

Beispiel E
Stimulation der Äthylenbiosynthese
Lösungsmittel:
30 Gewichtsteile Dimethylformamid
Emulgator:
1 Gewichtsteil Polyoxyäthylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Aus Sojabohnenblättern werden Blattstücke gleicher Grösse gestanzt. In Petrischalen, die mit 10 ml der Wirkstoffzubereitungen bzw. entsprechender Kontroll-Lösungen ohne Wirkstoffe gefüllt sind, wird jeweils eine konstante Zahl Blattstücke 1 Stunde lang inkubiert. Anschliessend werden die Blattstücke in luftdicht abgeschlossene Gefässe gegeben. Ein Teil der Wirkstoffzubereitungen wird ebenfalls in diese Gefässe zugegeben. Nach 24 Stunden wird das Äthylen, das sich in den Gefässen gesammelt hat, mit üblichen Nachweismethoden bestimmt. Die Äthylenentwicklung der mit Wirkstoffzubereitungen behandelten Blattstücke wird mit der Äthylenentwicklung der Kontrollen verglichen.

Das Pflanzenhormon Äthylen greift in zahlreiche Prozesse bei der Entwicklung der Pflanzen ein. Eine Erhöhung der Äthylenbiosynthese, wie sie mit den erfindungsgemässen Substanzen erzielt werden kann, erlaubt es, diese Prozesse zu steuern. Als Beispiele, für die besonders ein kommerzielles Interesse besteht, seien hier genannt: Fruchtablösung, Reifebeschleunigung von Früchten und Blättern, Blühinduktion, Samenkeimung, Fruchtausdünnung, Stimulation des Latexflusses z.B. bei Hevea und Wuchshemmung z.B. auch um das Lagern von Getreide zu verhindern.

Die Wirkstoffe und die Resultate gehen aus der nachfolgenden Tabelle hervor:

Tabelle E
Stimulation der Äthylenbiosynthese

| Wirkstoff | Wirkung |
|---|---|
| — (Kontrolle) | keine Aktivität |
| 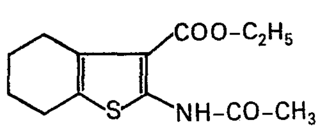 (bekannt) | keine Aktivität |

Tabelle E (Fortsetzung)
Stimulation der Äthylenbiosynthese

| Wirkstoff | Wirkung |
|---|---|
| 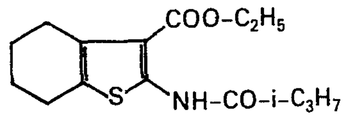 (bekannt) | keine Aktivität |
| 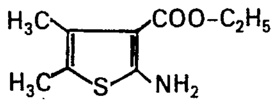 (2) | Aktivität |
| 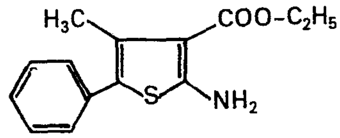 (1) | Aktivität |
| 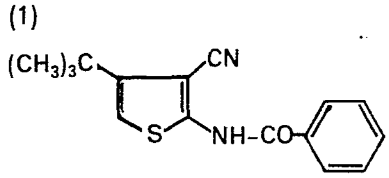 (27) | Aktivität |
| 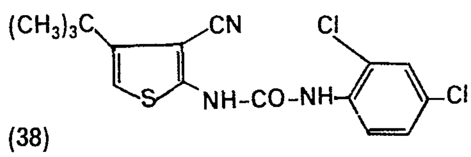 (38) | Aktivität |
| 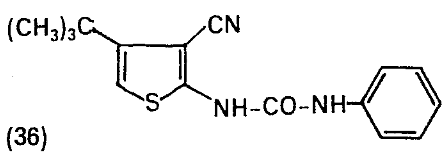 (36) | Aktivität |
| 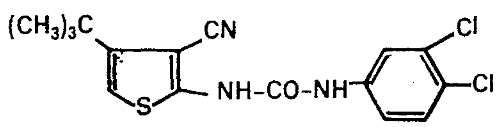 (39) | Aktivität |
| 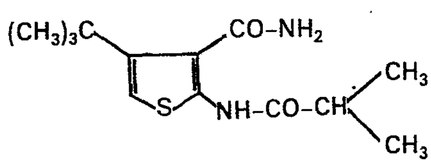 (43) | Aktivität |

65

Tabelle E (Fortsetzung
Stimulation der Äthylenbiosynthese

| Wirkstoff | Wirkung |
|---|---|
| 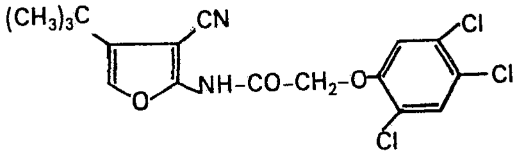 (62) | Aktivität |
| 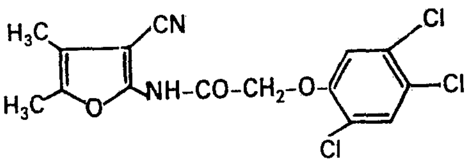 (55) | Aktivität |
| 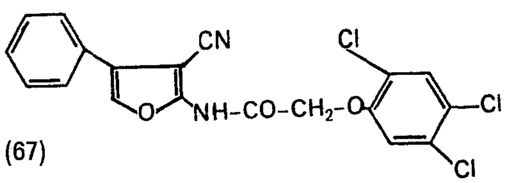 (67) | Aktivität |
| 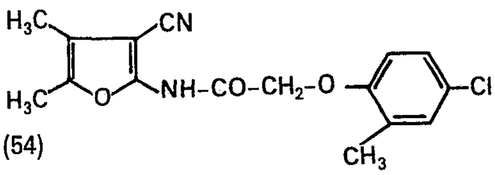 (54) | Aktivität |
| 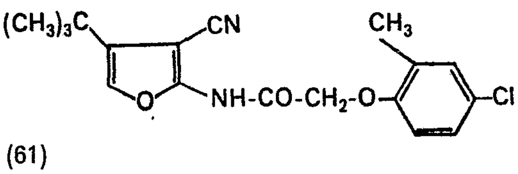 (61) | Aktivität |
| 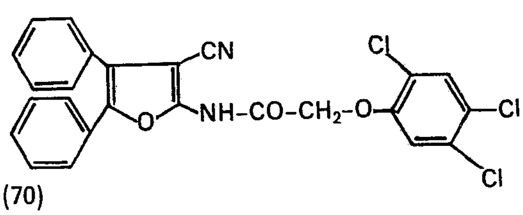 (70) | Aktivität |

Beispiel F

Wuchshemmung bei Baumwolle

Lösungsmittel:
30 Gewichtsteile Dimethylformamid

Emulgator:
1 Gewichtsteil Polyoxyäthylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Laubblattes angezogen. In diesem Stadium werden die Pflanzen tropfnass mit den Wirkstoffzubereitungen besprüht. Nach 3 Wochen wird bei allen Pflanzen der Zuwachs gemessen und die Wuchshemmung in % des Zuwachses der Kontrollpflanzen berechnet. Es bedeuten 100 % den Stillstand des Wachstums und 0 % ein Wachstum entsprechend dem der Kontrollpflanzen.

Wirkstoffe, Wirkstoffkonzentrationen und Resultate gehen aus der nachfolgenden Tabelle hervor.

Tabelle F
Wuchshemmung bei Baumwolle

| Wirkstoff | Wirkstoff-konzentration in % | Wuchs-hemmung in % |
|---|---|---|
| — (Kontrolle) | — | 0 |
| 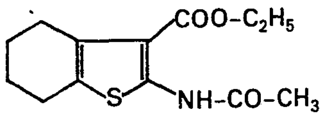 (bekannt) | 0,05 | 0 |
| 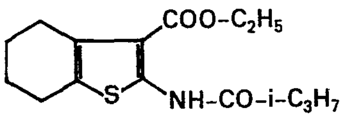 (bekannt) | 0,05 | 0 |
| 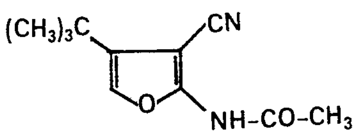 (71) | 0,05 | 50 |
| 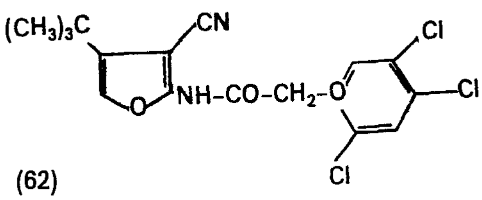 (62) | 0,05 | 95 |
| 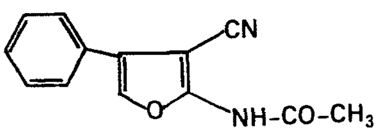 (73) | 0,05 | 60 |
| 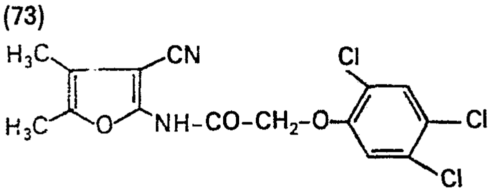 (55) | 0,05 | 95 |
| 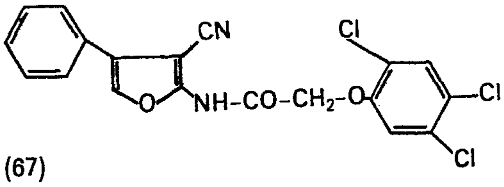 (67) | 0,05 | 95 |
| 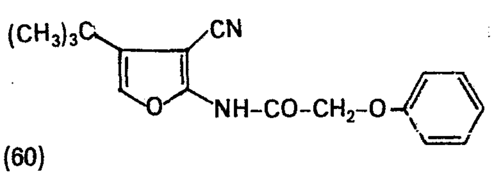 (60) | 0,05 | 100 |

Tabelle F (Fortsetzung)
Wuchshemmung bei Baumwolle

| Wirkstoff | Wirkstoff-konzentration in % | Wuchs-hemmung in % |
|---|---|---|
| 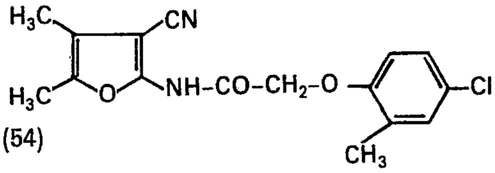 (54) | 0,05 | 100 |
| 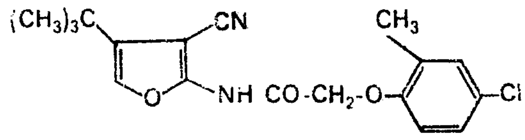 (61) | 0,05 | 80 |
| 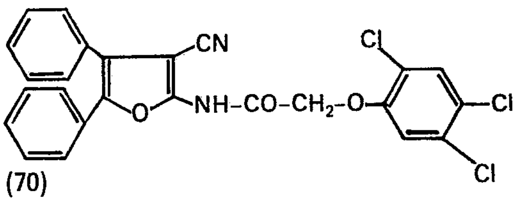 (70) | 0,05 | 90 |

Beispiel G

Wuchshemmung bei Sojabohnen

Lösungsmittel:
30 Gewichtsteile Dimethylformamid
Emulgator:
1 Gewichtsteil Polyoxyäthylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnass mit denWirkstoffzubereitungen besprüht. Nach 3 Wochen wird bei allen Pflanzen der Zuwachs gemessen und die Wuchshemmung in % des Zuwachses der Kontrollpflanzen berechnet. Es bedeuten 100% den Stillstand des Wachstums und 0 % ein Wachstum entsprechend dem der Kontrollpflanzen.

Wirkstoffe, Wirkstoffkonzentrationen und Resultate gehen aus der nachfolgenden Tabelle hervor.

Tabelle G
Wuchshemmung bei Sojabohnen

| Wirkstoff | Wirkstoff-konzentration in % | Wuchs-hemmung in % |
|---|---|---|
| — (Kontrolle) | — | 0 |
| $Cl-CH_2-CH_2-\overset{\oplus}{N}(CH_3)_3 \overset{\ominus}{Cl}$ (bekannt) | 0,05 | 0 |
| 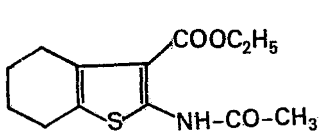 (bekannt) | 0,05 | 0 |

Tabelle G (Fortsetzung)
Wuchshemmung bei Sojabohnen

| Wirkstoff | Wirkstoff-konzentration in % | Wuchs-hemmung in % |
|---|---|---|
| 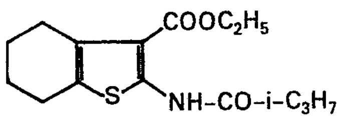 (bekannt) | 0,05 | 0 |
| 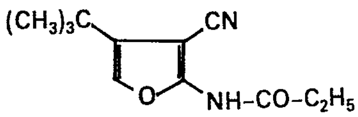 (72) | 0,05 | 25 |
| 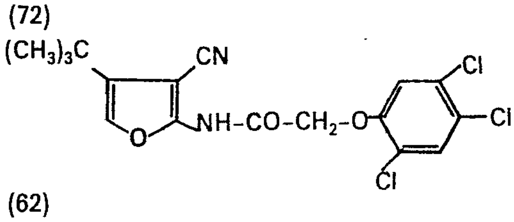 (62) | 0,05 | 95 |
| 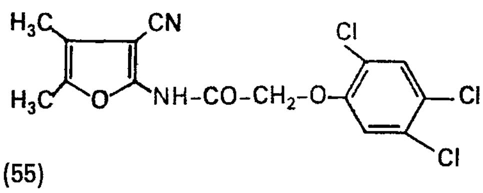 (55) | 0,05 | 100 |
| 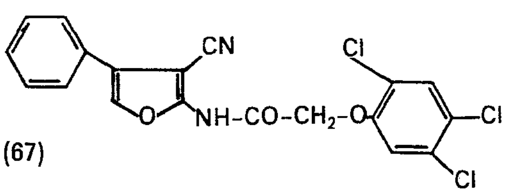 (67) | 0,05 | 100 |
| 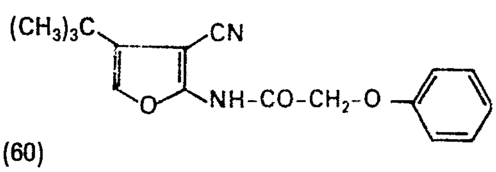 (60) | 0,05 | 20 |
| 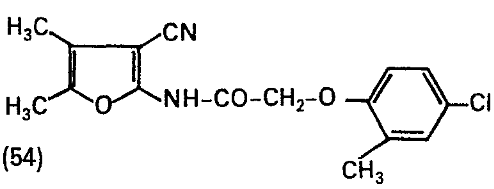 (54) | 0,05 | 100 |
| 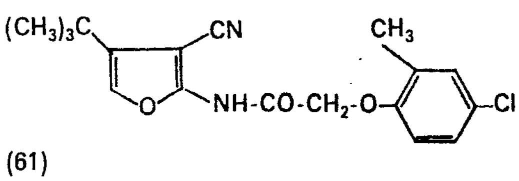 (61) | 0,05 | 90 |

Tabelle G (Fortsetzung)
Wuchshemmung bei Sojabohnen

| Wirkstoff | Wirkstoff-konzentration in % | Wuchs-hemmung in % |
|---|---|---|
| 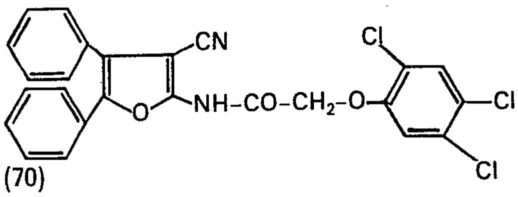 (70) | 0,05 | 90 |

Beispiel H

Entlaubung und Desiccation bei Baumwolle

Lösungsmittel:
30 Gewichtsteile Dimethylformamid

Emulgator:
1 Gewichtsteil Polyoxyäthylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Laubblattes angezogen. In diesem Stadium werden die Pflanzen tropfnass mit den Wirkstoffzubereitungen besprüht.

Nach 1 Woche werden der durch die Präparate induzierte Blattfall und die Austrocknung der Blätter (Desiccation) bonitiert. Die Ergebnisse werden mit denen der unbehandelten Kontrollpflanzen verglichen.

Der Grad der Entlaubung und der Desiccation wird bestimmt und mit Kennziffern von 0–3 bezeichnet, welche folgende Bedeutung haben:

0 = keine Wirkung (wie unbehandelte Kontrolle)
1 = schwache Wirkung
2 = mittlere Wirkung
3 = starke Wirkung

Wirkstoffe, Wirkstoffkonzentrationen und Resultate gehen aus der nachfolgenden Tabelle hervor.

Tabelle H
Entlaubung und Desiccation bei Baumwolle

| Wirkstoff | Wirkstoff-konzentration in % | Entlaubung und Desiccation |
|---|---|---|
| — (Kontrolle) | — | 0 |
| 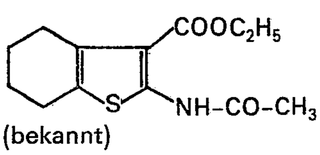 (bekannt) | 0,05 | 0 |
| 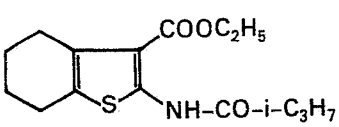 (bekannt) | 0,05 | 0 |
| 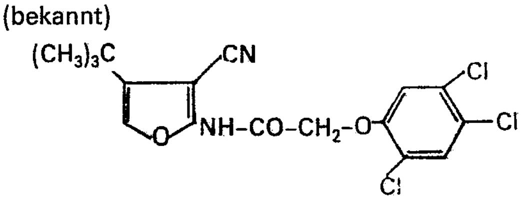 (62) | 0,05 | 1 |

Tabelle H (Fortsetzung)
Entlaubung und Desiccation bei Baumwolle

| Wirkstoff | Wirkstoff-konzentration in % | Entlaubung und Desiccation |
|---|---|---|
| 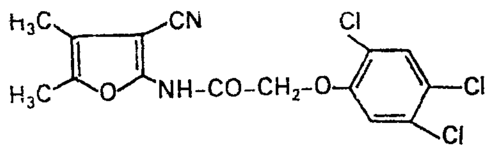 (55) | 0,05 | 2 |
| 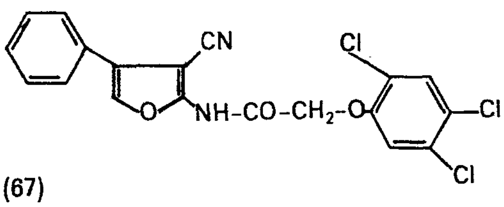 (67) | 0,05 | 2 |
| 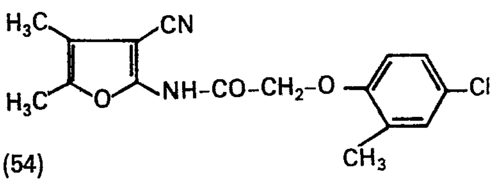 (54) | 0,05 | 2 |
| 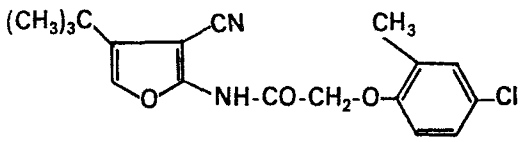 (61) | 0,05 | 1 |
| 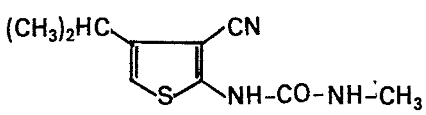 (32) | 0,05 | 3 |
| 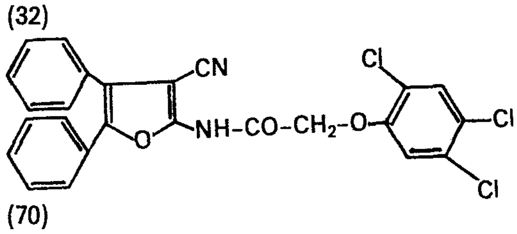 (70) | 0,05 | 1 |

Herstellungsbeispiele

Beispiel 1

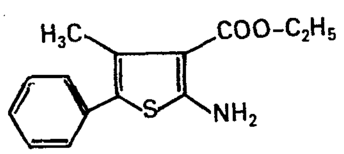

In eine Mischung aus 201 g (1,5 Mol) Methylbenzylketon, 204 g (1,8 Mol) Cyanessigsäureäthylester und 48 g (1,5 Mol) Schwefel in 100 ml Äthanol werden unter Rühren 131 g (1,5 Mol) Morpholin gegeben. Es tritt eine exotherme Reaktion ein, wobei man die Temperatur auf 60°C ansteigen lässt. Man lässt das Reaktionsgemisch über Nacht stehen, giesst dann in Wasser, nimmt das sich abscheidende Öl in Methylenchlorid auf und wäscht die organische Phase nacheinander mit Wasser und verdünnter Essigsäure. Nach dem Abziehen des Lösungsmittels kristallisiert man den teilweise erstarrenden Rückstand (387 g) aus Cyclohexan um. Man erhält auf diese Weise 166 g an 2-Amino-3-äthoxy-carbonyl-4-methyl-5-phenyl-thiophen in Form einer Festsubstanz vom Schmelzpunkt 93°C.

In analoger Weise werden die in der nachfolgenden Tabelle formelmässig aufgeführten Verbindungen hergestellt.

Tabelle 1

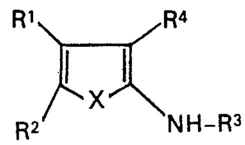

| Beispiel-Nr. | X | R[1] | R[2] | R[3] | R[4] | Schmelzpunkt [°C] |
|---|---|---|---|---|---|---|
| 2 | S | $CH_3$ | $CH_3$ | H | $-COO-C_2H_5$ | 92 |
| 3 | S | $i-C_4H_9$ | H | H | $-COOC_2H_5$ | 65 |
| 4 | S | 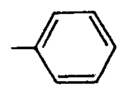 | H | H | $-COOC_2H_5$ | 96 |

Beispiel 5

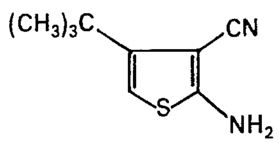

In ein Gemisch aus 1480 g (10 Mol) 1-tert.-Butyl-2,2-dicyan-äthylen und 320 g (10 Mol) Schwefel in 4 Litern Äthanol werden unter intensivem Rühren 870 ml (10 Mol) Morpholin getropft. Dabei wird durch anfängliches leichtes Erwärmen und spätere Kühlung eine Temperatur von 45 - 50°C eingehalten. Nach dem Abklingen der exothermen Reaktion rührt man noch 2 Stunden bei 50°C, zieht dann 3 Liter Äthanol unter vermindertem Druck ab und verrührt den Rückstand mit 5 Litern Eiswasser. Das dabei ausfallende Produkt wird abgesaugt, gewaschen und getrocknet. Man erhält auf diese Weise 1750 g an 2-Amino-3-cyano-4-tert.-butyl-thiophen vom Schmelzpunkt 92°C.

Das als Ausgangsprodukt verwendete 1-tert.-Butyl-2,2-dicyan-äthylen wird folgendermassen hergestellt:

Ein Gemisch aus 990 g (15 Mol) Malonsäuredinitril, 2625 g (26,25 Mol) Methyl-tert.-butyl-keton, 145 g (1,88 Mol) Ammoniumacetat, 225 g (3,75 Mol) Essigsäure und 2 Litern Toluol wird so lange am Wasserabscheider gekocht (10–13 Stunden), bis sich die abgeschiedene Wassermenge nicht mehr vermehrt. Man verdünnt die dunkelbraune Reaktionslösung mit 2 Litern Toluol, verrührt dann mit 5 Litern Wasser, trennt die Phasen, filtriert die organische Phase und dampft ein. Der verbleibende Rückstand wird unter vermindertem Druck einer fraktionierten Destillation unterworfen. Man erhält 1616 g 1-tert.-Butyl-2,2-dicyan-äthylen, das bei 0,2 Torr einen Siedepunkt von 70°C besitzt.

$n_D^{20} = 1,4798$

Gemäss Gaschromatogramm beträgt der Reinheitsgrad des Produkts 99 %.

Nach der im Beispiel 5 beschriebenen Methode werden auch die in den Beispielen 6 und 7 aufgeführten Verbindungen hergestellt.

Beispiel 6

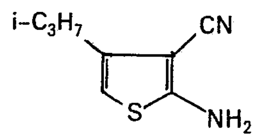

Schmelzpunkt 70°C

Ausgangsprodukt:

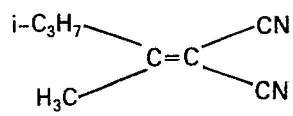

Sdp.: 65°C bei 0,15 Torr

$n_D^{20} = 1,4706$

Beispiel 7

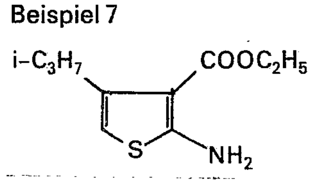

Schmelzpunkt 62°C

Ausgangsprodukt:

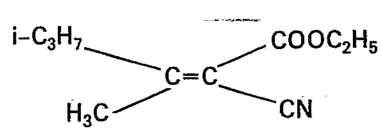

Sdp.: 69°C bei 0,2 Tor

$n_D^{20} = 1,4689$

Beispiel 8

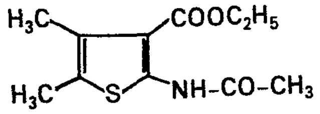

59,7 g (0,2 Mol) 2-Amino-3-äthoxycarbonyl-4,5-dimethyl-thiophen werden in 72 ml Essigsäure gelöst und mit 33,7 g (0,33 Mol) Acetanhydrid versetzt. Nach dem Abklingen der exothermen Reaktion giesst man das Reaktionsgemisch in 400 ml Eiswasser, saugt das ausfallende Produkt ab und wäscht mit Wasser. Man erhält auf diese Weise 69,7 g 2-Acetylamino-3-äthoxycarbonyl-4,5-dimethyl-thiophen vom Schmelzpunkt 98°C (nach Umkristallisation aus Cyclohexan).

Nach der im Beispiel 8 beschriebenen Methode werden auch die in der folgenden Tabelle formelmässig aufgeführten Verbindungen hergestellt.

Tabelle 2

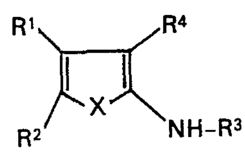

| Bsp.-Nr. | X | $R^1$ | $R^2$ | $R^3$ | $R^4$ | Schmelzpunkt [°C] |
|---|---|---|---|---|---|---|
| 9 | S | $CH_3$ | $CH_3$ | $CO-C_2H^5$ | $CO_2-C_2H_5$ | 73 |
| 10 | S | $CH_3$ | $CH_3$ | $CO-i-C_3H_7$ | $CO_2-C_2H_5$ | 46 |
| 11 | S | $CH_3$ | $CH_3$ | $CO-t-C_4H_9$ | $CO_2-C_2H_5$ | 81 |
| 12 | S | $i-C_4H_9$ | H | $CO-CH_3$ | $CO_2-C_2H_5$ | 62 |
| 13 | S | $i-C_4H_9$ | H | $CO-C_2H_5$ | $CO_2-C_2H_5$ | 39 |
| 14 | S | $i-C_4H_9$ | H | $CO-i-C_3H_7$ | $CO_2-C_2H_5$ | 34 |
| 15 | S | $i-C_4H_9$ | H | $CO-t-C_4H_9$ | $CO_2-C_2H_5$ | 67 |
| 16 | S | 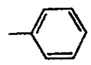 | H | $CO-CH_3$ | $CO_2-C_2H_5$ | 93 |
| 17 | S | 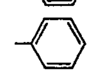 | H | $CO-C_2H_5$ | $CO_2-C_2H_5$ | 68 |
| 18 | S | 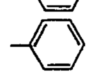 | H | $CO-i-C_3H_7$ | $CO_2-C_2H_5$ | 92 |
| 19 | S | 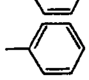 | H | $CO-t-C_4H_9$ | $CO_2-C_2H_5$ | 112 |
| 20 | S | $i-C_3H_7$ | H | $CO-CH_3$ | CN | 140 |
| 21 | S | $i-C-C_3H_7$ | H | $CO-C_2H_5$ | CN | 137 |
| 22 | S | $i-C_3H_7$ | H | $CO-CF_3$ | CN | 155 |
| 23 | S | $t-C_4H_9$ | H | $CO-CH_3$ | CN | 160 |
| 24 | S | $t-C_4H_9$ | H | $CO-DF_3$ | CN | 156 |
| 25 | S | $t-C_4H_9$ | H | $CO-C_2H_5$ | CN | 157 |
| 26 | S | $t-C_4H_9$ | H | $CO-i-C_3H_7$ | CN | 136 |

Beispiel 27

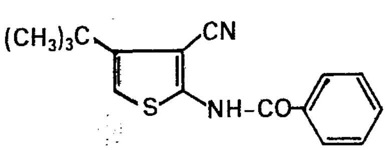

Ein Gemisch aus 36 g (0,2 Mol) 2-Amino-3-cyano-4-tert.-butyl-thiophen und 200 ml Toluol wird nach Zusatz von 29,4 g (0,21 Mol) Benzoylchlorid so lange gekocht (4 Stunden), bis die Chlorwasserstoff-Entwicklung beendet ist. Danach wird das Reaktionsgemisch filtriert und am Rotationsverdampfer eingeengt. Der verbleibende Rückstand wird aus Methanol umkristallisiert. Man erhält auf diese Weise 46,4 g 2-Benzoylamino-3-cyano-4-tert.-butyl-thiophen vom Schmelzpunkt 130°C.

Nach der im Beispiel 27 beschriebenen Methode werden auch die in der folgenden Tabelle formelmässig aufgeführten Verbindungen hergestellt.

Tabelle 3

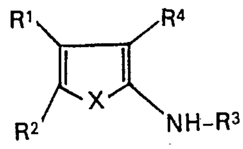

| Bsp.-Nr. | X | $R^1$ | $R^2$ | $R^3$ | $R^4$ | Schmelzpunkt [°C] |
|---|---|---|---|---|---|---|
| 28 | S | $t\text{-}C_4H_9$ | H | $CO\text{-}CH_2Cl$ | CN | 126 |
| 29 | S | $t\text{-}C_4H_9$ | H | $CO\text{-}t\text{-}C_4H_9$ | CN | 173 |
| 30 | S | $t\text{-}C_4H_9$ | H | $CO\text{-}O\text{-}CH_3$ | CN | 114 |
| 31 | S | $t\text{-}C_4H_9$ | H | CO-O- 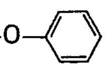 | CN | 125 |

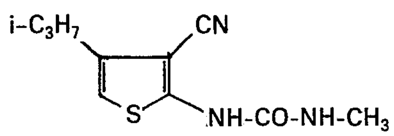

In eine Lösung von 33,2 g (0,2 Mol) 2-Amino-3-cyano-4-isopropyl-thiophen in 200 ml Toluol werden 11,8 ml (0,2 Mol) Methylisocyanat gegeben. Nach dem Abklingen der exothermen Reaktion wird das in fester Form anfallende Produkt abgesaugt. Weitere Anteile an Reaktionsprodukt werden durch Einengen der Mutterlauge gewonnen. Man erhält auf diese Weise 25 g an 1-(3-cyano-4-isopropyl-thienyl (2)-3-methyl-harnstoff vom Schmelzpunkt 179 - 181°C.

Nach der im Beispiel 32 beschriebenen Methode werden auch die in der folgenden Tabelle formelmässig aufgeführten Verbindungen hergestellt:

Tabelle 4

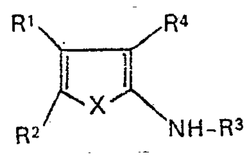

| Bsp.-Nr. | X | $R^1$ | $R^2$ | $R^3$ | $R^4$ | Schmelzpunkt [°C] |
|---|---|---|---|---|---|---|
| 33 | S | $t\text{-}C_4H_9$ | H | $CO\text{-}NH\text{-}CH_3$ | CN | 220 |
| 34 | S | $t\text{-}C_4H_9$ | H | $CO\text{-}NH\text{-}CH_2\text{-}CH{=}CH_2$ | CN | 150 |
| 35 | S | $t\text{-}C_4H_9$ | H | $CO\text{-}NH\text{-}n.\text{-}C_4H_9$ | CN | 157 |
| 36 | S | $t\text{-}C_4H_9$ | H | CO-NH- 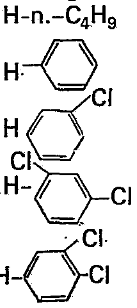 | CN | 202 |
| 37 | S | $t\text{-}C_4H_9$ | H | CO-NH | CN | 240 |
| 38 | S | $t\text{-}C_4H_9$ | H | CO-NH- | CN | 238 |
| 39 | S | $t\text{-}C_4H_9$ | H | CO-NH- | CN | 271 |

Beispiel 40

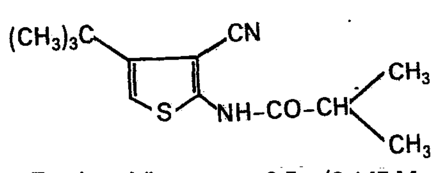

Zu einer Lösung von 6,5 g (0,145 Mol) Dimethylamin in 200 ml Toluol tropft man bei Raumtemperatur 20,6 g (0,1 Mol) 3-cyano-4-tert.-butyl-thienyl (2)-isocyanat. Nach Abklingen der exothermen Reaktion wird eingeengt und der verbleibende Rückstand aus wenig Toluol umkristallisiert. Man erhält auf diese Weise 20,1 g an 1-(3-Cyano-4-tert.-butyl-thienyl (2)-3,3-dimethyl-harnstoff vom Schmelzpunkt 140°C.

Herstellung des Ausgangsproduktes:

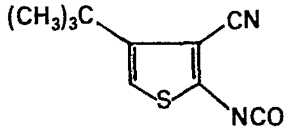

Zu einer Lösung von 150 g Phosgen in 1,5 Litern Chlorbenzol tropft man bei 0 - 5°C eine Lösung von 90 g (0,5 Mol) 2-Amino-3-cyano-4-tert.-butyl-thiophen in 300 ml Chlorbenzol. Danach heizt man unter Einleiten von weiterem Phosgen langsam bis zum Sieden und vertreibt dann überschüssiges Phosgen durch Einleiten von trockenem Stickstoff. Zur Aufarbeitung zieht man das Lösungsmittel unter vermindertem Druck ab und destilliert den Rückstand. Man erhält auf diese Weise 62 g an 3-Cyano-4-tert.-butyl-thienyl-(2)-isocyanat, das bei einem Druck von 20 Torr bei 163°C siedet.

Schmelzpunkt 61 - 64°C.

Beispiel 41

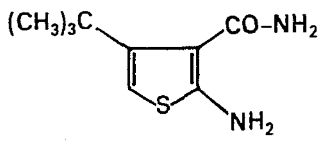

Ein Gemisch aus 180 g (1 Mol) 2-Amino-3-cyano-4-tert.-butyl-thiophen und 750 ml 95 - 97%ige Schwefelsäure wird eine Stunde lang bei 100 - 110°C gerührt, wobei eine leicht exotherme Reaktion einsetzt, so dass beim Erreichen einer Temperatur von 100°C nicht mehr geheizt zu werden braucht. Man lässt das Reaktionsgemisch abkühlen, rührt es in 5 Liter Eiswasser ein und neutralisiert mit etwa 3 Litern 25 %iger wässriger Ammoniak-Lösung. Das ausfallende Festprodukt wird abgesaugt, mit Wasser gewaschen und bei 50°C getrocknet. Man erhält auf diese Weise 194 g an 2-Amino-3-aminocarbonyl-4-tert.-butyl-thiophen vom Schmelzpunkt 145°C.

Beispiel 42

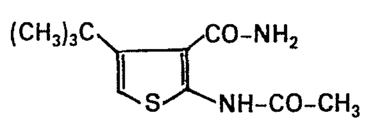

Zu einer Suspension von 39,6 g (0,2 Mol) 2-Amino-3-aminocarbonyl-4-tert.-butyl-thiophen in 300 ml Toluol gibt man zunächst 21,2 g (0,21 Mol) Triäthylamin und tropft dann 16,5 g (0,21 Mol) Acetylchlorid hinzu. Nach beendeter exothermer Reaktion wird aufgearbeitet, indem man das Festprodukt absaugt und mit Wasser wäscht. Man erhält auf diese Weise 38,6 g an 2-Acetyl-amino-3-amino-carbonyl-4-tert.- butyl-thiophen vom Schmelzpunkt 251°C.

Beispiel 43

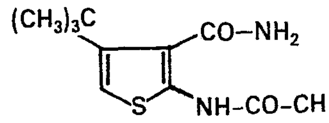

Zu einer Lösung von 39,6 g (0,2 Mol) 2-Amino-3-amino-carbonyl-4-tert.-butyl-thiophen in 200 ml Pyridin tropft man unter Kühlung bei Temperaturen zwischen 10°C und 30°C 22,2 g (0,21 Mol) Isobuttersäurechlorid. Nach 30 Minuten giesst man das Reaktionsgemisch in 2 Liter Eiswasser, saugt das ausfallende Festprodukt ab, wäscht es mit Wasser und trocknet bei 50°C. Man erhält auf diese Weise 42 g an 2-Isobutyrylamino-3-amino-carbonyl-4-tert.-butyl-thiophen, das nach Umkristallisation aus einem Dimethylformamid-Methanol-Gemisch einen Schmelzpunkt von 226°C besitzt.

Nach der im Beispiel 43 beschriebenen Methode wird auch die in dem folgenden Beispiel aufgeführte Verbindung hergestellt.

Beispiel 44

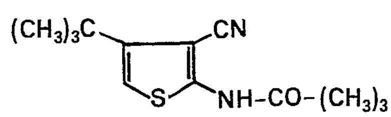

Schmelzpunkt 221°C

Beispiel 45

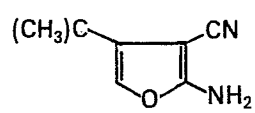

Zu einer Lösung von 232 g (2 Mol) Hydroxy-pinakolin und 132 g (2 Mol) Malonsäuredinitril in 500 ml Dimethylformamid tropft man unter Kühlung 174 g (2 Mol) Morpholin so hinzu, dass die Innentemperatur nicht über 40°C ansteigt. Nach beendeter Reaktion wird das Reaktionsgemisch in 5 Liter Eiswasser eingerührt. Das dabei ausfallende Festprodukt wird abgesaugt, mit Wasser gewaschen und getrocknet. Man erhält auf diese Weise 170 g an 2-Amino-3-cyano-4-tert.-butyl-furan vom Schmelzpunkt 86°C.

Nach der im Beispiel 45 beschriebenen Methode werden auf die in den folgenden Beispielen aufgeführten Verbindungen hergestellt.

Beispiel 46

Schmelzpunkt 167°C

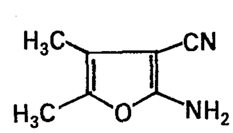

Beispiel 47

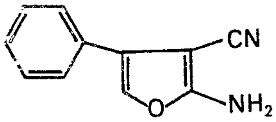

Schmelzpunkt 87°C

Beispiel 48

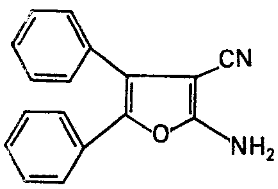

Schmelzpunkt 202°C

Beispiel 49

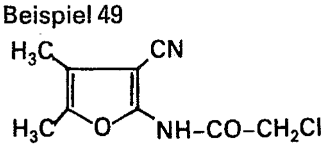

20,4 g (0,1 Mol) 2-Amino-3-cyano-4,5-dimethylfuran und 17,5 g (0,155 Mol) Chloracetylchlorid werden in 200 ml Toluol bis zum Abklingen der Chlorwasserstoff-Entwicklung gekocht. Danach wird das Lösungsmittel unter vermindertem Druck verdampft und der verbleibende Rückstand aus Essigester umkristallisiert. Man erhält auf diese Weise 26 g an 2-Chloracetylamino-3-cyano-4,5-dimethylfuran vom Schmelzpunkt 114°C.

Nach der im Beispiel 49 beschriebenen Methode werden auch die in der folgenden Tabelle formelmässig aufgeführten Verbindungen hergestellt.

Tabelle 5

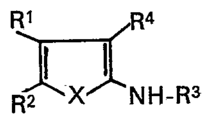

| Bsp.-Nr., | X | $R^1$ | $R^2$ | $R^3$ | $R^4$ | Schmelzpunkt [°C] |
|---|---|---|---|---|---|---|
| 50 | O | $CH_3$ | $CH_3$ | $CO-C_2H_5$ | CN | 97 |
| 51 | O | $CH_3$ | $CH_3$ | $CO-i-C_3H_7$ | CN | 150 |
| 52 | O | $CH_3$ | $CH_3$ | $CO-CH_2-O-$ 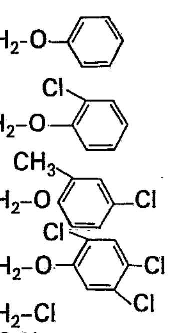 | CN | 126 |
| 53 | O | $CH_3$ | $CH_3$ | $CO-CH_2-O-$ | CN | 136 |
| 54 | O | $CH_3$ | $CH_3$ | $CO-CH_2-O$ | CN | 156 |
| 55 | O | $CH_3$ | $CH_3$ | $CO-CH_2-O-$ | CN | 179 |
| 56 | O | $t-C_4H_9$ | H | $CO-CH_2-Cl$ | CN | 83 |
| 57 | O | $t-C_4H_9$ | H | $CO-i-C_3H_7$ | CN | 117 |
| 58 | O | $t-C_4H_9$ | H | $CO-t-C_4H_9$ | CN | 160 |
| 59 | O | $t-C_4H_9$ | H | CO- 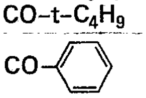 | CN | 139 |
| 60 | O | $t-C_4H_9$ | H | $CO-CH_2-O-$ 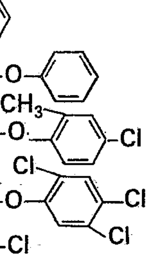 | CN | 128 |
| 61 | O | $t-C_4H_9$ | H | $CO-CH_2-O-$ | CN | 109 |
| 62 | O | $t-C_4H_9$ | H | $CO-CH_2-O-$ | CN | 170 |
| 63 | O | 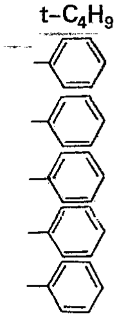 | H | $CO-CH_2-Cl$ | CN | 168 |
| 64 | O | | H | $CO-i-C_3H_7$ | CN | 148 |
| 65 | O | | H | $CO-t-C_4H_9$ | CN | 148 |
| 66 | O | | H | $CO-CH_2-O-$ 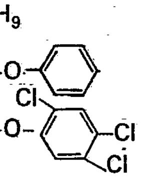 | CN | 156 |
| 67 | O | | H | $CO-CH_2-O-$ | CN | 204 |

Tabelle 5 (Fortsetzung)

| Bsp.-Nr. | X | R[1] | R[2] | R[3] | R[4] | Schmelzpunkt [°C] |
|---|---|---|---|---|---|---|
| 68 | O | 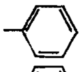 | 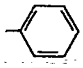 | CO-CH$_2$-Cl | CN | 192 |
| 69 | O | 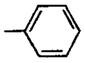 | 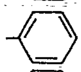 | CO-CH$_2$-O-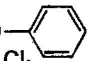 | CN | 126 |
| 70 | O | 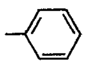 | 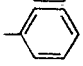 | CO-CH$_2$-O-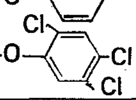 | CN | 214 |

Beispiel 71

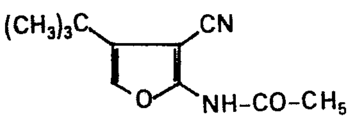

16,4 g (0,1 Mol) 2-Amino-3-cyano-4-tert.-butyl-furan werden mit 24 ml (0,4 Mol) Essigsäure und 10,4 ml (0,11 Mol) Essigsäureanhydrid eine Stunde lang auf 100°C erhitzt. Danach dampft man das Reaktionsgemisch unter vermindertem Druck ein und kristallisiert den verbleibenden Rückstand aus einem Gemisch von Toluol/Cyclohexan = 1:2 um. Man erhält auf diese Weise 17,2 g an 2-Acetylamino-3-cyano-4-tert.-butyl-furan vom Schmelzpunkt 135°C.

nach der im Beispiel 71 beschriebenen Methode werden auch die in den folgenden Beispielen aufgeführten Verbindungen hergestellt.

Beispiel 72

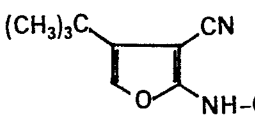

Schmelzpunkt 103°C

Beispiel 73

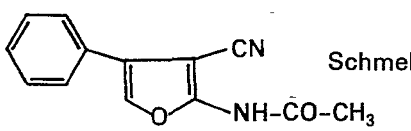

Schmelzpunkt 148°C

Beispiel 74

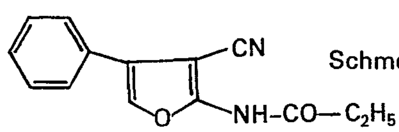

Schmelzpunkt 150°C

Beispiel 75

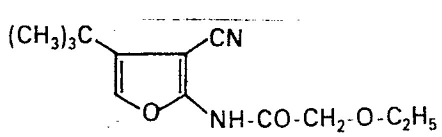

Zu einer Lösung von 32,8 g (0,2 Mol) 2-Amino-3-cyano-4-tert.-butyl-furan in 200 ml Dimethylformamid tropft man bei Raumtemperatur 26,2 g (0,2 Mol) Äthoxyacetylchlorid. Nach dem Abklingen der mässig exothermen Umsetzung (Temperaturanstieg von 23°C auf 38°C) wird das Reaktionsgemisch in Eiswasser gegossen. Das dabei anfallende Festprodukt wird abgesaugt und im feuchten Zustand aus wenig Methanol umkristallisiert. Man erhält auf diese Weise 39,3 g an 2-Äthoxyacetylamino-3-cyano-4-tert.-butyl-furan vom Schmelzpunkt 98°C.

Nach der im Beispiel 75 beschriebenen Methode werden auch die in den folgenden Beispielen aufgeführten Verbindungen hergestellt:

Beispiel 76

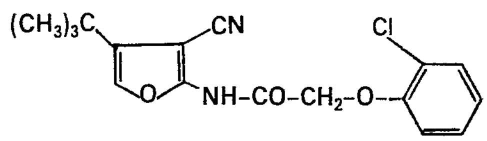

Schmelzpunkt 116°C

Beispiel 77

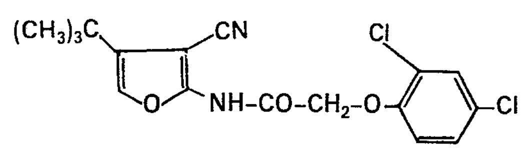

Schmelzpunkt 139°C

Beispiel 78

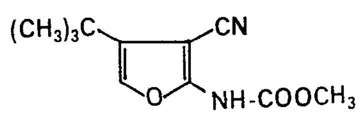

Schmelzpunkt 103°C

Beispiel 79

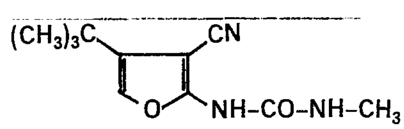

Eine Lösung von 7,8 g (0,041 Mol) 3-Cyano-4-tert.-butyl-furyl-(2)-isocyanat in 30 ml Toluol wird zu einer Lösung von 1,6 g (0,052 Mol) Methylamin in 30 ml Toluol getropft. Danach arbeitet man auf, indem man das Reaktionsgemisch unter vermindertem Druck einengt und den verbleibenden Rückstand aus Toluol umkristallisiert. Man erhält auf diese Weise 6,8 g an 1-(3-Cyano-4-tert.-butyl-furyl(2)-3-methylharnstoff vom Schmelzpunkt 163°C.

Das als Ausgangsprodukt benötigte 3-Cyano-4-tert.-butyl-furyl(2)-isocyanat wird durch Phosgenierung von 2-Amino-3-cyano-4-tert.-butyl-furan hergestellt.

Schmelzpunkt 39 - 41°C
Siedepunkt 71°C bei 0,6 Torr

Nach der im Beispiel 79 beschriebenen Methode werden auch die in den folgenden Beispielen aufgeführten Verbindungen hergestellt.

Beispiel 80

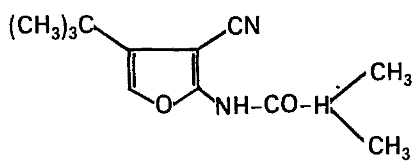

Schmelzpunkt 155°C

Beispiel 81

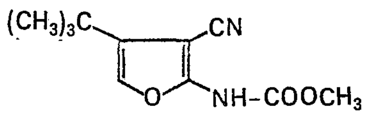

Schmelzpunkt 103°C.

**Patentanspruch**

Verwendung von Furan- und Thiophenderivaten der Formel

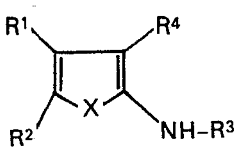

(I)

in welcher

$R^1$ für Alkyl mit 1 bis 6 Kohlenstoffatomen oder für gegebenenfalls durch Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen, Alkylthio mit 1 bis 4 Kohlenstoffatomen, Alkylsulfonyl mit 1 bis 4 Kohlenstoffatomen und/oder Nitro substituiertes Phenyl oder Naphthyl steht,

$R^2$ für Wasserstoff, Alkyl mit 1 bis 6 Kohlenstoffatomen oder für gegebenenfalls durch Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen, Alkylthio mit 1 bis 4 Kohlenstoffatomen, Alkylsulfonyl mit 1 bis 4 Kohlenstoffatomen und/oder Nitro substituiertes Phenyl oder Naphthyl steht,

$R^3$ für Wasserstoff oder die Gruppierung

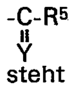

steht

in welcher

Y für Sauerstoff oder Schwefel steht und

$R^5$ für Alkyl mit 1 bis 6 Kohlenstoffatomen, Halogenalkyl mit 1 bis 4 Kohlenstoffatomen und 1 bis 5 Halogenatomen, Alkoxyalkyl mit 1 bis 4 Kohlenstoffatomen im Alkylteil und 1 bis 4 Kohlenstoffatomen im Alkoxyteil, gegebenenfalls im Aryloxyteil durch Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen, Alkylthio mit 1 bis 4 Kohlenstoffatomen, Alkylsulfonyl mit 1 bis 4 Kohlenstoffatomen und/oder Nitro substituiertes Aryloxyalkyl mit 6 oder 10 Kohlenstoffatomen im Aryloxyteil und 1 bis 4 Kohlenstoffatomen im Alkylteil, für Alkoxy mit 1 bis 4 Kohlenstoffatomen, gegebenenfalls durch Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen, Alkylthio mit 1 bis 4 Kohlenstoffatomen, Alkylsulfonyl mit 1 bis 4 Kohlenstoffatomen und/oder Nitro substituiertes Phenyl oder Naphthyl, für gegebenenfalls durch Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen, Alkylthio mit 1 bis 4 Kohlenstoffatomen, Alkylsulfonyl mit 1 bis 4 Kohlenstoffatomen und/oder Nitro substituiertes Phenoxy oder Naphthoxy, für Monoalkylamino mit 1 bis 4 Kohlenstoffatomen, Dialkylamino mit 1 bis 4 Kohlenstoffatomen in jeder Alkylgruppe, Alkenylamino mit bis zu 4 Kohlenstoffatomen, Dialkenylamino mit bis zu 4 Kohlenstoffatomen in jeder Alkenylgruppe, für gegebenenfalls durch Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen, Alkylthio mit 1 bis 4 Kohlenstoffatomen, Alkylsulfonyl mit 1 bis 4 Kohlenstoffatomen und/oder Nitro substituiertes Arylamino mit 6 oder 10 Kohlenstoffatomen, für einen 5- bis 7-gliedrigen gesättigten heterocyclischen Ring, der über Stickstoff gebunden ist, und in dem 1 bis 3 Kohlenstoffatome ersetzt sein können durch Sauerstoff, Schwefel, $-SO_2-$, -NH- und/oder $-N(CH_3)-$, oder für die Gruppierung der Formel

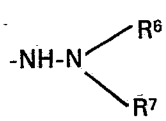

steht,

in welcher

$R^6$ für Wasserstoff oder Alkyl mit 1 bis 4 Kohlenstoffatomen steht,

$R^7$ für Wasserstoff oder Alkyl mit 1 bis 4 Kohlen-

stoffatomen steht und
$R^6$ und $R^7$ gemeinsam mit dem angrenzenden Stickstoffatom für einen 5- bis 7-gliedrigen gesättigten heterocyclischen Ring stehen, in dem 1 bis 3 Kohlenstoffatome ersetzt sein können durch Sauerstoff, Schwefel, $-SO_2-$, -NH- und/oder $-N(CH_3)-$,
$R^4$ für Cyano oder die Gruppierung $-COR^8$ steht, in welcher
$R^8$ für Alkoxy mit 1 bis 4 Kohlenstoffatomen oder Amino steht, und
X für Sauerstoff oder Schwefel steht,
zur Hemmung des Pflanzenwuchses, zur Stimulation der pflanzlichen Ethylenbiosynthese, zur Entblätterung bei Baumwolle sowie zur Reifebeschleunigung bei Tomaten.

**Patent Claim**

Use of furan and thiophene derivatives of the formula

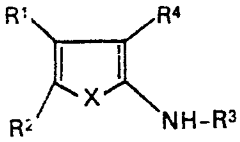

(I)

in which
$R^1$ represents alkyl with 1 to 6 carbon atoms; or phenyl or naphthyl each optionally substituted by halogen, alkyl with 1 to 4 carbon atoms, halogenoalkyl with 1 to 4 carbon atoms and 1 to 5 halogen atoms, alkoxy with 1 to 4 carbon atoms, alkylthio with 1 to 4 carbon atoms, alkylsulphonyl with 1 to 4 carbon atoms and/or nitro,
$R^2$ represents hydrogen, alkyl with 1 to 6 carbon atoms; or phenyl or naphthyl each optionally substituted by halogen, alkyl with 1 to 4 carbon atoms, halogenoalkyl with 1 to 4 carbon atoms and 1 to 5 halogen atoms, alkoxy with 1 to 4 carbon atoms, alkylthio with 1 to 4 carbon atoms, alkylsulphonyl with 1 to 4 carbon atoms and/or nitro, $R^3$ represents hydrogen or the grouping

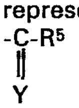

in which
Y represents oxygen or sulphur and
$R^5$ represents alkyl with 1 to 6 carbon atoms; halogenoalkyl with 1 to 4 carbon atoms and 1 to 5 halogen atoms; alkoxyalkyl with 1 to 4 carbon atoms in the alkyl part and 1 to 4 carbon atoms in the alkoxy part; aryloxyalkyl with 6 or 10 carbon atoms in the aryloxy part and 1 to 4 carbon atoms in the alkyl part and which is optionally substituted in the aryloxy part by halogen, alkyl with 1 to 4 carbon atoms, halogenoalkyl with 1 to 4 carbon atoms and 1 to 5 halogen atoms, alkoxy with 1 to 4 carbon atoms, alkylthio with 1 to 4 carbon atoms, alkylsulphonyl with 1 to 4 carbon atoms and/or nitro; alkoxy with 1 to 4 carbon atoms; phenyl or naphthyl each optionally substituted by halogen, alkyl with 1 to 4 carbon atoms, halogenoalkyl with 1 to 4 carbon atoms and 1 to 5 halogen atoms, alkoxy with 1 to 4 carbon atoms, alkylthio with 1 to 4 carbon atoms, alkylsulphonyl with 1 to 4 carbon atoms and/or nitro; phenoxy or naphthoxy each optionally substituted by halogen, alkyl with 1 to 4 carbon atoms, halogenoalkyl with 1 to 4 carbon atoms and 1 to 5 halogen atoms, alkoxy with 1 to 4 carbon atoms, alkylthio with 1 to 4 carbon atoms, alkylsulphonyl with 1 to 4 carbon atoms and/or nitro; monoalkylamino with 1 to 4 carbon atoms, dialkylamino with 1 to 4 carbon atoms in each alkyl group, alkenylamino with up to 4 carbon atoms, dialkenylamino with up to 4 carbon atoms in each alkenyl group, arylamino with 6 or 10 carbon atoms which is optionally substituted by halogen, alkyl with 1 to 4 carbon atoms, halogenoalkyl with 1 to 4 carbon atoms and 1 to 5 halogen atoms, alkoxy with 1 to 4 carbon atoms, alkylthio with 1 to 4 carbon atoms, alkylsulphonyl with 1 to 4 carbon atoms and/or nitro; a 5-membered to 7-membered saturated heterocyclic ring which is bonded via nitrogen and in which 1 to 3 carbon atoms can be replaced by oxygen, sulphur, $-SO_2-$, -NH- and/or $-N(CH_3)-$; or the grouping of the formula

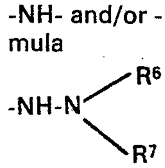

in which
$R^6$ represents hydrogen or alkyl with 1 to 4 carbon atoms,
$R^7$ represents hydrogen or alkyl with 1 to 4 carbon atoms and
$R^6$ and $R^7$, together with the adjacent nitrogen atom, represent a 5-membered to 7-membered saturated heterocyclic ring in which 1 to 3 carbon atoms can be replaced by oxygen, sulphur, $-SO_2-$, -NH- and/or $-N(CH_3)-$,
$R^4$ represents cyano or the grouping $-COR^8$,
in which
$R^8$ represents alkoxy with 1 to 4 carbon atoms or amino, and
X represents oxygen or sulphur,
for inhibiting plant growth, for stimulating ethylene biosynthesis in plants, for defoliation in the case of cotton and for accelerating ripening in the case of tomatoes.

**Revendication**

Utilisation de dérivés de furanne et de thiophène pour l'inhibition de la croissance des plantes, pour la stimulation de la biosynthèse végétale d'éthylène, pour la défoliation du coton, de même que pour l'accélération de la maturation des tomates, ces dérivés de furanne et de thiophène répondant à la formule:

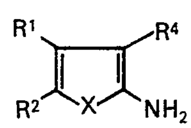

(I)

dans laquelle
$R^1$ représente un groupe alkyle contenant 1 à 6 atomes de carbone ou un groupe naphtyle ou un groupe phényle éventuellement substitué par un

atome d'halogène, par un groupe alkyle contenant 1 à 4 atomes de carbone, par un groupe halogénoalkyle contenant 1 à 4 atomes de carbone et 1 à 5 atomes d'halogènes, par un groupe alcoxy contenant 1 à 4 atomes de carbone, par un groupe alkylthio contenant 1 à 4 atomes de carbone, par un groupe alkylsulfonyle contenant 1 à 4 atomes de carbone et/ou par un groupe nitro,

$R^2$ représente un atome d'hydrogène, un groupe alkyle contenant 1 à 6 atomes de carbone ou un groupe naphtyle ou un groupe phényle éventuellement substitué par un atome d'halogène, par un groupe alkyle contenant 1 à 4 atomes de carbone, par un groupe halogénoalkyle contenant 1 à 4 atomes de carbone et 1 à 5 atomes d'halogènes, par un groupe alcoxy contenant 1 à 4 atomes de carbone, par un groupe alkylthio contenant 1 à 4 atomes de carbone, par un groupe alkylsulfonyle contenant 1 à 4 atomes de carbone et/ou par un groupe nitro,

$R^3$ représente un atome d'hydrogène ou le groupement

dans lequel

Y représente un atome d'oxygène ou un atome de soufre, et

$R^5$ représente un groupe alkyle contenant 1 à 6 atomes de carbone, un groupe halogénoalkyle contenant 1 à 4 atomes de carbone et 1 à 5 atomes d'halogènes, un groupe alcoxyalkyle contenant 1 à 4 atomes de carbone dans la fraction alkyle et 1 à 4 atomes de carbone dans la fraction alcoxy, un groupe aryloxy-alkyle contenant 6 ou 10 atomes de carbone dans la fraction aryloxy et 1 à 4 atomes de carbone dans la fraction alkyle et éventuellement substitué dans la fraction aryloxy par un atome d'halogène, par un groupe alkyle contenant 1 à 4 atomes de carbone, par un groupe halogénoalkyle contenant 1 à 4 atomes de carbone et 1 à 5 atomes d'halogènes, par un groupe alcoxy contenant 1 à 4 atomes de carbone, par un groupe alkylthio contenant 1 à 4 atomes de carbone, par un groupe alkylsulfonyle contenant 1 à 4 atomes de carbone et/ou par un groupe nitro, un groupe alcoxy contenant 1 à 4 atomes de carbone, un groupe naphtyle ou un groupe phényle éventuellement substitué par un atome d'halogène, par un groupe alkyle contenant 1 à 4 atomes de carbone, par un groupe halogénoalkyle contenant 1 à 4 atomes de carbone et 1 à 5 atomes d'halogènes, par un groupe alcoxy contenant 1 à 4 atomes de carbone, par un groupe alkylthio contenant 1 à 4 atomes de carbone, par un groupe alkylsulfonyle contenant 1 à 4 atomes de carbone et/ou par un groupe nitro, un groupe naphtoxy ou phénoxy éventuellement substitué par un atome d'halogène, par un groupe alkyle contenant 1 à 4 atomes de carbone, par un groupe halogénoalkyle contenant 1 à 4 atomes de carbone et 1 à 5 atomes d'halogènes, par un groupe alcoxy contenant 1 à 4 atomes de carbone, par un groupe alkylthio contenant 1 à 4 atomes de carbone, par un groupe alkylsulfonyle contenant 1 à 4 atomes de carbone et/ou par un groupe nitro, un groupe monoalkylamino contenant 1 à 4 atomes de carbone, un groupe dialkylamino contenant 1 à 4 atomes de carbone dans chaque groupe alkyle, un groupe alcénylamino contenant jusqu'à 4 atomes de carbone, un groupe dialcénylamino contenant jusqu'à 4 atomes de carbone dans chaque groupe alcényle, un groupe arylamino contenant 6 ou 10 atomes de carbone et éventuellement substitué par un atome d'halogène, par un groupe alkyle contenant 1 à 4 atomes de carbone, par un groupe halogénoalkyle contenant 1 à 4 atomes de carbone et 1 à 5 atomes d'halogènes, par un groupe alcoxy contenant 1 à 4 atomes de carbone, par un groupe alkylthio contenant 1 à 4 atomes de carbone, par un groupe alkylsulfonyle contenant 1 à 4 atomes de carbone et/ou par un groupe nitro, un noyau hétérocyclique pentagonal à heptagonal saturé, relié par un atome d'azote et dans lequel 1 à 3 atomes de carbone peuvent être remplacés par un atome d'oxygène, par un atome de soufre, par un groupe $-SO_2-$, par un groupe -NH- et/ou par un groupe $-N(CH_3)-$,

ou le groupement de formule

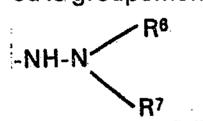

dans lequel

$R^6$ représente un atome d'hydrogène ou un groupe alkyle contenant 1 à 4 atomes de carbone,

$R^7$ représente un atome d'hydrogène ou un groupe alkyle contenant 1 à 4 atomes de carbone, et

$R^6$ et $R^7$, ensemble avec l'atome d'azote adjacent, représentent un noyau hétérocyclique pentagonal à heptagonal saturé dans lequel 1 à 3 atomes de carbone peuvent être remplacés par un atome d'oxygène, par un atome de soufre, par un groupe $-SO_2-$, par un groupe -NH- et/ou par un groupe $-N(CH)_3-$,

$R^4$ représente un groupe cyano ou le groupement $-COR^8$, dans lequel

$R^8$ représente un groupe alcoxy contenant 1 à 4 atomes de carbone, ou un groupe amino, et

X représente un atome d'oxygène ou de soufre.